(19) Europäisches Patentamt
European Patent Office
Office européen des brevets

(11) **EP 3 949 923 B1**

(12) # EUROPEAN PATENT SPECIFICATION

(45) Date of publication and mention
of the grant of the patent:
**17.12.2025 Bulletin 2025/51**

(21) Application number: **20814691.0**

(22) Date of filing: **25.05.2020**

(51) International Patent Classification (IPC):
*A61F 13/494* *(2006.01)*    *A61F 13/49* *(2006.01)*
*A61F 13/532* *(2006.01)*    *A61F 13/535* *(2006.01)*

(52) Cooperative Patent Classification (CPC):
**A61F 13/49413; A61F 13/4906; A61F 13/4942;
A61F 13/532; A61F 13/535;** A61F 2013/49433;
A61F 2013/5355

(86) International application number:
**PCT/JP2020/020514**

(87) International publication number:
**WO 2020/241559 (03.12.2020 Gazette 2020/49)**

---

(54) **DISPOSABLE DIAPER**

WEGWERFWINDEL

COUCHE JETABLE

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2019 JP 2019102943**

(43) Date of publication of application:
**09.02.2022 Bulletin 2022/06**

(73) Proprietor: **Kao Corporation
Chuo-ku
Tokyo 103-8210 (JP)**

(72) Inventors:
• **FUJINAKA, Tomoko
Haga-gun, Tochigi 321-3497 (JP)**
• **OKUDA, Yasuyuki
Haga-gun, Tochigi 321-3497 (JP)**

(74) Representative: **Vossius & Partner
Patentanwälte Rechtsanwälte mbB
Siebertstraße 3
81675 München (DE)**

(56) References cited:
**EP-A1- 1 880 700        JP-A- 2003 265 529
JP-A- 2016 077 880        JP-A- 2016 112 208
JP-A- H10 504 987         JP-A- H11 299 828
US-A1- 2005 113 790**

---

**Description**

Technical Field

The present invention relates to a disposable diaper, such as a disposable pull-on diaper.

Background Art

Disposable diapers generally have a leak-proof cuff attached along laterally opposite, longitudinally extending sides of an absorbent member to provide protection against leakage of bodily discharges. With a view to improving the feel of the leak-proof cuff against the skin, various studies have been directed to the structure of the sheet material forming the leak-proof cuffs.

For example, JP 2011-177549A listed below discloses use of nonwoven fabric made up of fine fibers with a prescribed diameter and having a prescribed basis weight, a prescribed air permeability value, and a prescribed moisture breathability value as a nonwoven material forming leak-proof cuffs. JP 2017-023782A teaches that, in the formation of a leak-proof cuff made of gathered nonwoven fabric which rises on contraction of an elastic member, the free edge portion of the standing cuff is embossed to be softened. JP 2019-010348A proposes using, as a material forming leak-proof cuffs, nonwoven fabric characterized in terms of basis weight, bending stiffness and bending hysteresis measured with a bending tester, softness and smoothness measured with a tissue softness tester, and water pressure resistance measured with a water pressure resistance tester.

Further related art is shown in US 2005/113790 A1, JP 2016 077880 A, or EP 1 880 700 A1

Summary of Invention

The present invention relates to a disposable diaper including an absorbent assembly including: a liquid permeable topsheet and an absorbent member; and a leak-proof cuff disposed along each of the sides of the absorbent assembly, the disposable diaper having a longitudinal direction, and a lateral direction perpendicular to the longitudinal direction, and the absorbent assembly having laterally opposite, longitudinally extending sides and side edges.

In an embodiment, the leak-proof cuff has opposite lateral side edges and opposite longitudinal ends, includes two facing nonwoven layers of nonwoven fabric (hereinafter also referred to as nonwoven layers), and has a free edge along one of the lateral side edges and a fixed edge along the other side edge.

In an embodiment, the leak-proof cuff includes: a fixed end portion fixed to the absorbent assembly and located at the longitudinal ends of the leak-proof cuff; an elasticized free edge region having an elastic member arranged along the free edge; and a fold located between the free edge and the fixed edge, the nonwoven fabric having an area located between the fixed edge and the elasticized free edge region, the area having a lateral compression load value of 5 cN or less.

In an embodiment, the leak-proof cuff has: an upper soft region including the two nonwoven layers and being located between the elasticized free edge region and the fold; and a lower joined region including the two nonwoven layers joined together partially or entirely and being located between the fold and the fixed edge.

In an embodiment, the two nonwoven layers of the upper soft region are unjoined together or have a lower peel strength than the two nonwoven layers of the lower joined region.

Brief Description of Drawings

[Fig. 1] Fig. 1 is a schematic perspective illustrating an embodiment of the disposable diaper of the invention.
[Fig. 2] Fig. 2 is a schematic plan of the skin facing surface side (interior surface side) of the diaper shown in Fig. 1 in its flat-out, uncontracted configuration.
[Fig. 3] Fig. 3 is a schematic cross-section taken along line III-III of Fig. 2.
[Fig. 4] Fig. 4 is a schematic cross-section of the crotch portion of the diaper shown in Fig. 1 in a worn state (corresponding to Fig. 3).
[Fig. 5] Fig. 5 is an enlarged plan view schematically illustrating the surface of the lower joined region of a leak-proof cuff.
[Fig. 6] Fig. 6 is an enlarged plan view schematically illustrating the surface of the upper soft region of a leak-proof cuff.
[Fig. 7] Fig. 7 is an enlarged schematic cross-section of a leak-proof cuff.

Description of Embodiments

When the soft nonwoven fabric disclosed in JP 2011-177549A, JP 2017-023782A, and JP 2019-010348A is used to make leak-proof cuffs with the view to improving the feel to the touch of leak-proof cuffs, the resulting leak-proof cuff tends to have insufficient upstanding capabilities due to the low stiffness of the nonwoven fabric, resulting in a failure to conform to the wearer's movement during wear. As a result, the diaper has a reduced fit to the skin to allow leakage of bodily waste.

In the light of the above circumstances, the invention provides a disposable diaper of which the leak-proof cuffs are formed of soften nonwoven fabric and yet have good upstanding capabilities to provide a good fit to the wearer's skin while worn.

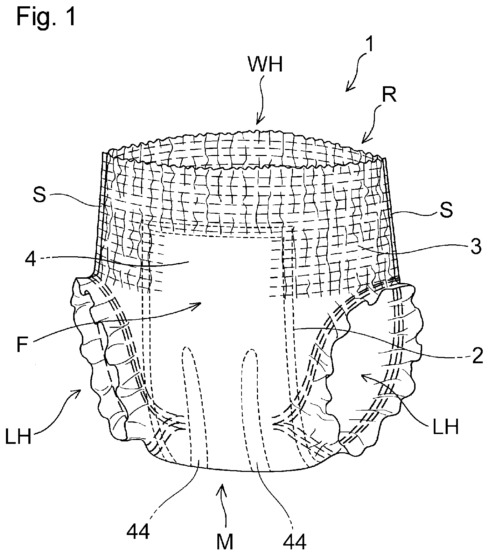

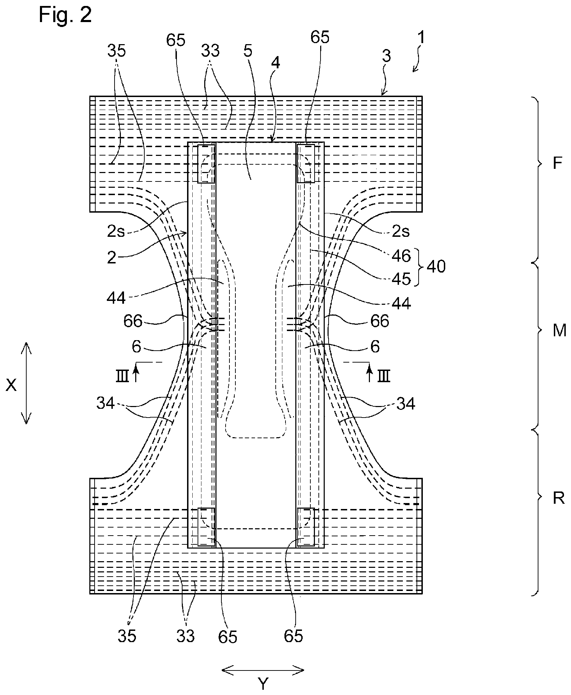

The invention will be described on the basis of its preferred embodiments with reference to the accompanying drawings. Figs. 1 and 2 illustrate a disposable pull-on diaper 1 according to an embodiment of the invention. As illustrated in Figs. 1 and 2, the diaper 1 includes a front portion F adapted to be worn about the front of a wearer, a rear portion R adapted to be worn about the back of the wearer, and a crotch portion M intermediate between the front portion F and the rear portions R. The front and rear portions F, R are connected to each other to form a tubular lower torso portion adapted to be worn about the wearer's lower torso. The diaper 1 has a longitudinal direction X corresponding to the wearer's front-to-back direction, i.e., the direction from the front portion to the crotch portion M to the rear portion R and a lateral direction Y perpendicular to the longitudinal direction X. The diaper 1 includes an absorbent assembly 2 and an outer cover 3 disposed on the non-skin facing surface side of the absorbent assembly 2.

As used herein, the term "skin facing surface" refers to the side of a disposable diaper or a member constituting the diaper (e.g., the absorbent assembly) facing the wearer's skin while worn, i.e., the surface relatively closer to the wearer's skin. The term "non-skin facing surface" refers to the side of a disposable diaper or a member constituting the diaper facing away from the wearer's skin while worn, i.e., the surface relatively farther from the wearer's skin. As used herein, the expression "while worn" and its analogues means the state of a disposable diaper maintained in an ordinarily worn state, i.e., in the right position on the body.

The front portion F and the rear portion M of the diaper 1 are joined together along their lateral side edges to form the outer cover 3 by a joining means, such as adhesive bonding, heat sealing, or ultrasonic sealing. The diaper 1 is of pull-on type as illustrated in Fig. 1. The diaper 1 has a pair of side seams S, a waist opening WH for receiving the waist of a wearer, and a pair of leg openings LH for receiving the legs of the wearer.

When the diaper 1 in a flat-out, uncontracted configuration is sectioned into equal thirds in the longitudinal direction X, the front, rear, and crotch portion F, R, and M correspond to the thirds as can be seen from Fig. 2. The absorbent assembly 2 of the diaper 1 shown in Fig. 2 is generally rectangular longer in the longitudinal direction X. The absorbent assembly 2 straddles the front portion F and the rear portion R. As used herein, the expression "flat-out, uncontracted configuration" (of the diaper 1) means a state in which the diaper 1 is opened by cutting apart the side seams S and with every elastic member stretched out until the diaper is straightened up to its design dimension with any influences of elastic members eliminated. The absorbent assembly 2 is located in the lateral middle of the outer cover 3. The absorbent assembly 2 is joined to the outer cover 3 by a joining means, such as adhesive bonding.

The absorbent assembly 2 is, as illustrated in Fig. 2, includes a liquid retentive absorbent member 4 and a liquid permeable topsheet 5 providing the skin facing surface of the diaper 1. The absorbent member 4 and the topsheet 5 are joined to each other by a known joining means, such as adhesive bonding. The absorbent member 4 is longer in the longitudinal direction X of the diaper 1 similarly to the absorbent assembly 2.

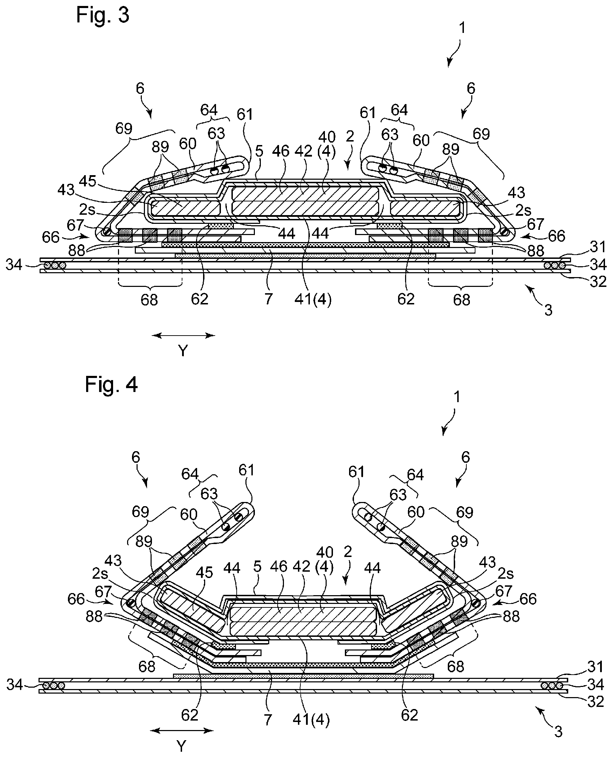

As illustrated in Figs. 2 and 3, the absorbent member 4 includes an absorbent core 40 and a core wrap sheet 41 covering the absorbent core 40. The absorbent core 40 is formed of a fiber aggregate that may have an absorbent polymer dispersed therein. The core wrap sheet 41 is formed of tissue or water-permeable nonwoven fabric. The core wrap sheet 41 preferably covers the entire area of the skin facing surface and the non-skin facing surface of the absorbent core 40.

The absorbent core 40 has a multi-layer structure as illustrated in Fig. 3. In more detail, the absorbent core 40 has a dual layer structure composed of a rectangular lower absorbent layer 45 and an upper absorbent layer 46 stacked on part of the lower absorbent layer 45. The absorbent core 40 includes a central portion 42 in the lateral middle of the crotch portion M. The central portion 42 is formed by stacking the upper absorbent layer 46 on the lower absorbent layer 45. The absorbent core 40 includes a pair of side portions 43 laterally opposite relative to the central portion 42. There is no upper absorbent layer 46 stacked on the lower absorbent layer 45 in the side portions 43. Therefore, the central portion 42 is thicker than the side portions 43. The absorbent core 40 may have a single layer structure.

The absorbent core 40 may or may not have a flexure region in the inside in the lateral direction. The flexure region may be a slit formed through the thickness of the absorbent core 40 or a low basis weight region having a lower basis weight than the surroundings. As illustrated in Figs. 2 and 3, the absorbent core 40 of the present embodiment has a pair of slits 44 extending in the longitudinal direction X as a flexure region. The slits 44 are located laterally inward of the longitudinally extending side edges 2s of the absorbent core 40 and laterally outward of the stack of the lower absorbent layer 45 and the upper absorbent layer 46 in the crotch portion M. The low basis weight region which may be provided in place of the slit 44 preferably has a basis weight of 10 to 220 g/m$^2$, more preferably 10 to 180 g/m$^2$.

The topsheet 5 is disposed on the skin facing surface side of the absorbent member 4. As illustrated in Fig. 3, the topsheet 5 covers the entire area of the skin facing surface side of the absorbent member 4 and wraps around both longitudinally-extending side edges to the non-skin facing surface side to cover the non-skin facing surface side of the side portions 43 of the absorbent member 4.

The absorbent assembly 2 is covered on its non-skin facing surface side with a liquid impermeable leak-proof sheet 7 formed of a resin film as illustrated in Fig. 3. The term "liquid impermeable" as used with respect to the leak-proof sheet 7 is intended to include "sparingly liquid permeable". The leak-proof sheet 7 is placed to cover the entire area of the non-skin facing surface of the absorbent member 4. To the skin facing surface of the outer cover 3 are joined the leak-proof sheet 7, nonwoven fabric 60 that forms the leak-proof cuffs 6, and the absorbent assembly 2 in the order described with an adhesive, such as a hot-melt adhesive.

The outer cover 3 of the diaper 1 is made up of two outer cover-forming sheets 31, 32 and elastic members fixed in their stretched state between the two sheets as illustrated in Fig. 3. As illustrated in Fig. 2, waist elastic members 33 forming waist gathers along the circumferential edge of the waist opening WH, leg elastic members 34 forming leg gathers along the circumferential edge of the leg openings LH, and lower torso elastic members 35 forming lower torso gathers in a lower torso region are arranged between the two sheets 31, 32. The lower torso region is a region from 20 mm below the edge of the waist opening WH to the upper end of the leg openings LH. The elastic members 33, 34, and 35 are each fixedly joined in a stretched state between the sheets 31, 32 by any joining means, such as a hot-melt adhesive.

As illustrated in Fig. 2, the absorbent assembly 2 has a pair of leak-proof cuffs 6 provided on its skin facing surface in the opposite, longitudinally extending lateral side portions thereof. Each leak-proof cuff 6 is formed of highly pliable nonwoven fabric in view of upstanding capabilities and flexibility to provide a good fit to the body of a wearer. The pliability of the nonwoven fabric 60 can be evaluated in terms of a compression load value. The nonwoven fabric 60 preferably has a lateral compression load value of 5 cN or less, more preferably 4 cN or less. The closer to zero the compression load value, the better, but a compression load value of 1 cN or more will be enough for sufficient pliability. The lateral compression load value of the nonwoven fabric 60 can be measured as follows.

Method for determining compression load value:

A leak-proof cuff 6 is cut off the absorbent assembly 2. A 20 mm wide sample having a length of 80 mm along the longitudinal direction is cut out from the nonwoven fabric 60 within a region between the opposing front and rear fixed portions 6F, 6R, at which the elasticized free edge region 64 having the leak-proof cuff elastic member fixed therein is fixed to the topsheet 5. When two facing layers of the nonwoven fabric are joined to each other, they are separated apart using a solvent or whatever to prepare a single sheet. The sample is folded lengthwise in two and again folded lengthwise in two to make a 4-layered specimen. When the leak-proof cuff has a region where two layers of the nonwoven fabric are joined with a 5 mm-wide lateral edge portion thereof remaining unjoined continuously in the longitudinal direction, a sample measuring 40 mm by 20 mm may be cut out of that region and folded lengthwise in two to prepare a 4-layered specimen. In that case, the non-bonded side of the specimen is subjected to the measurement. The specimen is smoothed out, and the 4 layers of the non-joined edge portion is clamped with a bulldog clip or something with their edges aligned. The specimen (width: 20 mm) is set with the direction Y vertical and the unjoined edge portion up and clamped by the lower jaw of a compression tester (e.g., Autograph AG-X from Shimadzu Corp.) such that the 5-mm wide upper edges project upward from the lower jaw. A 30 mm diameter circular compression plate attached above the specimen compresses the specimen at a rate of 10 mm/min to record the maximum load.

The dimension of the specimen in the lateral direction Y may be 20 mm or less as long as it can be clamped so as to project upward by 5 mm from the lower jaw.

Five samples are tested, and the average of the measurements is taken as the compression load value of the nonwoven fabric.

The leak-proof cuff 6 of the embodiment shown in Fig. 3 is formed of a single sheet of nonwoven fabric 60 folded in two layers facing each other.

The leak-proof cuff 6 has a free edge 61 and a fixed edge 62 along opposite lateral side edges. The free edge 61 contains one or more elastic member 63 adhesively fixed between the facing layers of the nonwoven fabric 60. Having the elastic member 63 fixed in its stretched state along the longitudinal direction X, the leak-proof cuff 6 is provided with the elasticized free edge region 64 arranged along the free edge 61. As illustrated in Fig. 3, the edge portion opposite to the free edge 61 of the leak-proof cuff 6 is joined to the part of the topsheet 5 that is wrapped around to the non-skin facing surface of the side portion 43 of the absorbent member 4 to form the fixed edge 62. The joining of the fixed edge 62 can be achieved by a known bonding method, such as heat sealing, ultrasonic sealing, or hot-melt adhesive bonding. The fixed edge 62 extends in parallel to the side edge 2s of the absorbent assembly 2.

As illustrated in Fig. 2, the leak-proof cuff 6 has a fixed end portion 65 fixed to the topsheet 5 of the absorbent assembly 2. The fixed end portion is located at longitudinal ends of the leak-proof cuff 6. The longitudinally opposite end portions of each leak-proof cuff 6, one in the front portion F and the other in the rear portion R, that include the free edge 61 are fixed to the topsheet 5 to form the fixed end portions 65. The leak-proof cuff 6 is configured to upstand in the crotch portion M during wear upon contraction of the elastic members 63 fixed in their stretched state as depicted in Fig. 4. The upstanding leak-proof cuffs 6 prevent bodily waste, such as urine, from running laterally outward.

As illustrated in Figs. 2 and 3, the leak-proof cuff 6 in a flat-out, uncontracted state has a fold 66. The leak-proof cuff 6 is folded along the fold 66. The fold 66 is located between the free edge 61 and the fixed edge 62. The part of the leak-proof cuff 6 between the free edge 61 and the fold 66 is folded over the skin facing surface of the absorbent assembly 2. The fold 66 is located laterally outward of the free edge 61. The so folded leak-proof cuff 6 is of what we call inward fold type.

The leak-proof cuff 6 may or may not have an elastic member along the fold 66. **In** the embodiment shown in Fig. 3, the leak-proof cuff 6 has one elastic member 67 along the fold 66 to help the leak-proof cuff 6 to bend easily. The elastic member 67 is adhesively fixed in its stretched state along the longitudinal direction X between the two layers of the nonwoven fabric 60.

As illustrated in Fig. 3, the leak-proof cuff 6 includes a lower joined region 68 between the fold 66 and the fixed edge 62 where the two layers of the nonwoven fabric 60 are partially or entirely joined to each other. The lower joined region 68 extends in the longitudinal direction X from the front portion F to the rear portion R.

As illustrated in Fig. 3, the leak-proof cuff 6 includes an upper soft region 69 formed of the two layers of the nonwoven fabric 60 and located between the elasticized free edge region 64 and the fold 66. Specifically, the upper soft region 69 is located between the laterally proximal edge of the elasticized free edge region 64 and the fold 66. When the proximal edge of the elasticized free edge region 64 is indistinct, the region between the elastic member 63 of the elasticized free edge region 64 and the fold 66 is defined to be the upper soft region 69. When there is a plurality of elastic members 63 providing the elasticized free edge region 64, the upper soft region 69 is a region between the most laterally proximal elastic member 63 and the fold 66. The upper soft region 69 extends in the longitudinal direction X between the front portion F and the rear portion R.

The diaper 1 of the present embodiment has a pleasant feel to the skin since the region between the elasticized free edge region 64 and the fixed edge 62 is formed of the soft nonwoven fabric 60 having a compression load value of as low as 5 cN or less. As illustrated in Figs. 3 and 4, the leak-proof cuff 6 has a lower joined region 68 formed of two layers of the nonwoven fabric 60 joined to each other. The lower joined region 68 thus provides the lower portion of the leak-proof cuff 6 from the fold 66 to the fixed edge 62 with stiffness. As a result, while the leak-proof cuff 6 is formed of the soft nonwoven fabric 60, it exhibits good upstanding capabilities.

The two layers of the nonwoven fabric 60 in the lower joined region 68 are fusion bonded in parts to form fusion bonds, whereby the leak-proof cuff 6 has improved resistance to water pressure to provide effective protection against leakage.

As illustrated in Figs. 3 and 4, the leak-proof cuff 6 has the upper soft region 69 between the elasticized free edge region 64 and the fold 66. The upper soft region 69 is formed of two layers of the nonwoven fabric 60 remaining unjoined to each other or joined together with a lower peel strength than the two layers of the nonwoven fabric 60 of the lower joined region 69. The upper soft region 69 thus maintains the essential pliability of the soft nonwoven fabric 60. Therefore, the leak-proof cuff 6 provides a better fit to the skin owing to the upper soft region 69 conforming to the skin's movement in contact with the skin during wear. As such, the diaper 1 of the embodiment having the leak-proof cuff 6 including the lower joined region 68 and the upper soft region 69 provides a superior fit to the wearer's skin, with the leak-proof cuff 6 satisfying the conflicting requirements of soft feel to the touch and good upstanding capabilities.

To ensure the effect of the lower joined region 68 in providing good upstanding capabilities of the leak-proof cuff 6, the peel strength between the two layers of the nonwoven fabric 60 in the lower joined region 68 is preferably 1.0 N/30 mm or higher, more preferably 1.2 N/30 mm or higher, preferably 3.0 N/30 mm or lower, more preferably 2.8 N/30 mm or lower, specifically preferably 1.0 to 3.0 N/30 mm, more preferably 1.2 to 2.8 N/30 mm.

To ensure the conformability of the upper soft region 69 to the skin's movement in contact with the skin, the peel strength between the two layers of the nonwoven fabric 60 in the upper soft region 69 is preferably lower than 1.0 N/30 mm, more preferably 0.5 N/30 mm or lower. It is even more preferred for the two nonwoven layers to be unjoined. The closer to 0 N/30 mm the peel strength of the upper soft region 69, the better, but a peel strength of 0.1 N/30 mm or higher will be sufficiently low to show sufficient conformability to the skin.

The peel strength of the lower joined region 68 and the upper soft region 69 is measured by the method below.

A rectangular specimen measuring longer than 50 mm in the longitudinal direction X and 30 mm in the lateral direction Y is cut out from each of the lower joined region 68 and the upper soft region 69 of the leak-proof cuff 6 of an unused disposable diaper. The two layers of the nonwoven fabric 60 are peeled apart from one of the longitudinal ends, and the separated nonwoven layers are clamped in the jaws of a tensile tester (e.g., Tensilon RTC series, from A and D Co., Ltd.) in a T-configuration at an initial jaw separation of 50 mm. The jaws are further separated at a rate of 300 mm/min to record the maximum strength. The measurement is taken in triplicate, and the average maximum strength is defined to be an interlaminar peel strength in unit of N/30 mm.

To further improve the upstanding capabilities of the leak-proof cuff 6 by taking advantage of the stiffness of the absorbent member 4, it is preferred for the fixed edge 62 of the leak-proof cuff 6 be located laterally inward of the side edge 2s of the absorbent assembly 2 as illustrated in Fig. 3.

To make the side portions 43 of the absorbent member 4 bend upward during wear so as to prevent bodily waste from running laterally outward, it is preferred that an entire of or a part of the lower joined region 68 be located laterally inward of the side edge 2s of the absorbent assembly 2 as illustrated in Fig. 4.

The absorbent core 40 of the diaper 1 has the slits 44. In the diaper of that type, the fixed edge 62 of the leak-proof cuff 6 is preferably located substantially directly beneath the slit 44 as illustrated in Fig. 4 so as to ensure the side portions 43 of the absorbent member 4 bending upward.

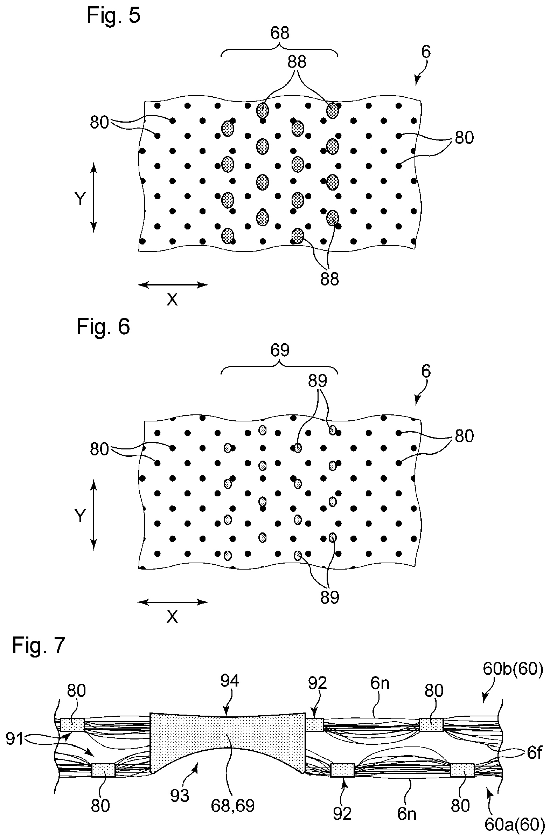

Among the upper soft region 69 and the lower joined region 68 of the leak-proof cuff 6, at least the lower joined region 68 preferably has fusion bonds 88 resulting from fusion bonding the two layers of the nonwoven fabric 60 in parts to impart stiffness to the lower portion of the leak-proof cuff 6 thereby to ensure good upstanding of the leak-proof cuff 6 as illustrated in Figs. 4 and 5.

It is preferred, from the viewpoint of helping the upper soft region 69 to smoothly move with the wearer's movement during wear, for the two layers of the nonwoven fabric 60 of the upper soft region 69 to remain unjoined to each other. The two layers of the nonwoven fabric 60 of the upper soft region 69 may be fusion bonded in parts to form fusion bonds 89 as illustrated in Figs. 4 and 6 so that the two nonwoven layers of the nonwoven fabric 60 may not be spaced from each other when the leak-proof cuff 6 is made to upstand by the contraction of the elastic member 63. As a result of the formation of the fusion bonds 89, the leak-proof cuff 6 is given moderate stiffness and thereby prevented from turning outboard, which will provide benefits for a caregiver in putting on the diaper.

To provide a pleasant feel to the skin, the nonwoven fabric 60 forming the leak-proof cuff 6 preferably has a mean deviation of coefficient of friction (MMD) of 0.015 or smaller. For the same purpose, the MMD of the nonwoven fabric 60 is more preferably 0.012 or smaller, even more preferably 0.010 or smaller. The closer to zero the MMD, the better, but an MMD of 0.003 or higher will be sufficiently small to ensure a sufficiently good feel to the skin.

To provide a good feel to the skin, the nonwoven fabric 60 forming the leak-proof cuff 6 preferably has a mean deviation of surface roughness (SMD) of 2.2 $\mu$m or smaller, more preferably 2.0 $\mu$m or smaller. The closer to zero the SMD, the better, but an SMD of 0.5 $\mu$m or higher will be sufficiently small to ensure a sufficiently good feel to the skin.

The MMD and SMD can be determined as follows using KESFB4-AUTO-A (trade name) from Kato Tech Co., Ltd. in accordance with the method described in Kawabata, Hideo, *fuai hyouka no hyoujunka to kaiseki* (Standardization and Analysis of Hand Evaluation), Japanese Hand Evaluation and Standardization Committee (HESC), The Textile Machinery Soc. of Japan, 2nd Ed. (Jul. 10, 1980).

Method for determining mean deviation of friction coefficient (MMD):

A sample measuring 20 cm by 20 cm is cut out of the nonwoven fabric forming the leak-proof cuff. When the nonwoven fabric being tested is short of that size, the size of the sample may be altered appropriately. The sample is clamped on a flat metal plate surface. A contact sensor is pressed onto the sample at a contact force of 49 cN, and the sample is moved horizontally at a constant speed of 0.1 cm/sec by 2 cm to be given a uniaxial tension of 7.3 cN/cm. The contact sensor is the same as one used for measuring the surface roughness, and is composed of a set of 20 piano wires of 0.5 mm in diameter arranged in parallel and U-shaped with a base width of 10 mm. The contact sensor is pressed onto the sample at a contact force of 49 cN using a weight. The mean deviation of friction coefficient is represented in terms of MMD value. The measurement is taken in both the MD and CD to obtain $MMD_{MD}$ and $MMD_{CD}$, respectively, an average of which obtained by the following formula (1) is taken as a mean deviation of friction coefficient MMD. In the case where the nonwoven fabric sheet is too small to make specimens for MD and CD measurements, either $MMD_{MD}$ or $MMD_{CD}$ is adopted. MD means the machine direction in the production of the nonwoven fabric or the longitudinal direction of the diaper, and CD means a direction perpendicular to the MD.

$$\text{Mean deviation of friction coefficient MMD} = \{(MMD_{MD}^2 + MMD_{CD}^2)/2\}^{1/2} \qquad (1)$$

Method for determining surface roughness mean deviation (SMD):

A sample measuring 20 cm by 20 cm is cut out of the nonwoven fabric forming the leak-proof cuff. When the nonwoven fabric being tested is short of that size, the size of the sample may be altered appropriately. The sample is clamped on a flat metal plate surface. A contact sensor is pressed onto the sample at a contact force of 9.8 cN (with an uncertainty of $\pm$0.49 cN), and the sample is moved horizontally at a constant speed of 0.1 cm/sec by 2 cm to be given a uniaxial tension of 7.3 cN/cm. The contact sensor is composed of a U-shaped single piano wire of 0.5 mm in diameter and 5 mm at the base. The contact sensor is urged onto the sample by a spring at a contact force of 9.8 cN. The spring had a spring constant of 24.5 cN/mm (with an uncertainty of $\pm$0.98 cN/mm) and a resonant frequency of at least 30 Hz out of contact with the sample surface. The mean deviation of surface roughness is represented in terms of SMD value. The measurement is taken in both the MD and CD to obtain $SMD_{MD}$ and $SNM_{CD}$, respectively, an average of which obtained by the following formula (2) is taken as a mean deviation of surface roughness SMD. In the case where the nonwoven fabric sheet is too small to make specimens for MD and CD measurements, either $SMD_{MD}$ or $SMD_{CD}$ is adopted. The MD and CD are as defined above.

$$\text{Mean deviation of surface roughness SMD} = \{(SMD_{MD}^2 + SMD_{CD}^2)/2\}^{1/2} \qquad (2)$$

Each leak-proof cuff 6 is formed of two facing layers of the nonwoven fabric 60 having an MMD of 0.015 or less. The facing nonwoven layers may be formed of different types of nonwoven fabrics that meet the MMD requirement or of a single sheet of nonwoven fabric that meets the MMD requirement folded in two.

With a view to imparting to the leak-proof cuff 6 cushioning properties created by surface unevenness, the nonwoven fabric 60 may preferably have embosses 80, where the constituent fibers are united, the embosses 80 being formed by embossing in a discrete pattern in plan view as shown in Figs. 5 and 6. In view of improved feel to the touch, the nonwoven fabric 60 preferably contains as a main component of constituent fibers a propylene random copolymer composed of propylene and an $\alpha$-olefin other than propylene. Examples of suitable nonwoven fabrics include spun-bonded, melt-blown, and spun-bonded/melt-blown laminated nonwoven fabric. With a view to improving the feel to the skin, the nonwoven fabric may contain a softening agent, such as a surfactant.

Referring to Fig. 7, each of the two facing layers of the nonwoven fabric 60 has a facing surface 6f that faces the opposing layer and a non-facing surface 6n on the opposite side, and each emboss 80 has a larger depth on the facing surface 6f than on the non-facing surface 6n with the view of improving the feel of the leak-proof cuff 6 to the touch. As illustrated in Fig. 7, the individual embosses 80 of each nonwoven layer have a depression 91 on the facing surface 6f and a depression 92 on the non-facing surface 6n, and the depression 91 is preferably deeper than the depression 92. The term "depression 91 or 92" means a depression having the emboss 80 as a bottom. The depth of the depressions of the individual embosses 80 can be measured by the following method.

Method for measuring depth of depression of emboss 80:

The nonwoven fabric 60 is cut using a knife, cutter, razor, and so on in the thickness direction along a line passing through a emboss 80, taking care not to deform the cut surface of the emboss 80. The cut surface is observed to measure the thicknesses of the emboss 80 and the adjacent, non-depressed portion on either side, and the difference in thickness is defined to be the depth of the emboss 80. The measurement may be made using, for example, Microscope VHX-1000 from Keyence Corp. fitted with a middle-range zoom lens at a tilt angle of 90°. The measurement is taken at ten embosses 80, and the average of the results is taken as the depth of the embosses 80.

The fusion bonds 88 and 89 can be formed by heat embossing, high-frequency sealing, ultrasonic sealing, and the like. Heat embossing is recommended for forming fusion bonds with a smaller thickness and a better feel against the skin. To improve the feel of the leak-proof cuff 6, as illustrated in Fig. 7, the fusion bonds 88 and 89 each provide a depression 93 on the nonwoven layer 60a facing the absorbent assembly and a depression 94 on the other nonwoven layer 60b such that the depression 93 is deeper than the depression 94. Such depressions 93 and 94 can be formed by performing heat embossing from the side of the nonwoven layer 60a. From the viewpoint of the balance between ease of processing and peel strength, the individual fusion bonds 88 preferably have a rectangular shape extending in the longitudinal direction X or, as illustrated in Fig. 5, an elongated circular shape extending in the longitudinal direction X, or a like shape.

With a view to preventing bodily waste from striking through the leak-proof cuff 6, the nonwoven fabric 60 preferably has a water pressure resistance of 130 mm or higher, more preferably 150 mm or higher. The higher the water pressure resistance, the better, but an upper limit of the water pressure resistance would be 250 mm for the leak-proof cuff 6 to exhibit sufficient protection against leakage. The water pressure resistance is represented in terms of water level (mm) in a water pressure resistance test. The water pressure resistance is measured by a water pressure resistance test in accordance with JIS L1092 (hydrostatic pressure method, method B).

From the standpoint of softness and breathability of the leak-proof cuff 6, in the nonwoven fabric 60, the ratio of the total area of the embosses 80 to the surface area of one surface of the nonwoven fabric 60 is preferably 16% or less, more preferably 12% or less. The lower the total area ratio, the better, but a lower limit of the total area of the embosses 80 would be 5% or higher for obtaining sufficient softness and breathability.

From the standpoint of softness and breathability of the leak-proof cuff 6, in the leak-proof cuff 6, the ratio of the total area of the fusion bonds 89 to the surface area of the upper soft region 69 is preferably 5% or less, more preferably 4% or less. The lower the total area ratio, the better, but a lower limit of the ratio of the total area of the fusion bonds 89 would be 2% or higher for obtaining sufficient softness and breathability.

To obtain both upstanding capabilities and breathability of the leak-proof cuff 6, in the leak-proof cuff 6, the ratio of the total area of the fusion bonds 88 to the surface area of the lower joined region 68 is preferably higher than the above-described total area ratio of the fusion bonds 89. The total area ratio of the fusion bonds 88 is preferably 5% or less, more preferably 4.5% or less. The lower the total area ratio, the preferred, but a lower limit of the total area ratio of the fusion bonds 88 would be 2.5% or higher for obtaining sufficient upstanding capabilities and breathability.

The area of the individual fusion bonds 88 and 89 is preferably larger than the area of the individual embosses 80, whereby the fusion bonds 88 and 89 for joining the two layers of the nonwoven fabric 60 can be formed at locations other than the embosses 80 to secure sufficient peel strength between the nonwoven layers. In order for the lower joined region 68 to provide the leak-proof cuff 6 with upstanding capabilities and for the upper soft region 69 to provide the leak-proof cuff 6 with a soft feel, the individual fusion bonds 88 preferably have a larger area than the individual fusion bonds 89.

The area of the individual embosses 80 formed in the nonwoven fabric 60 is preferably $0.10 \text{ mm}^2$ or larger, more preferably $0.13 \text{ mm}^2$ or large, preferably $0.35 \text{ mm}^2$ or smaller, more preferably $0.32 \text{ mm}^2$ or smaller.

The area of the individual fusion bonds 88 formed in the lower joined region 68 is preferably $0.38 \text{ mm}^2$ or larger, more preferably $0.4 \text{ mm}^2$ or larger, preferably $3.0 \text{ mm}^2$ or smaller, more preferably $2.7 \text{ mm}^2$ or smaller.

The area of the individual fusion bonds 89 formed in the upper soft region 69 is preferably $0.35 \text{ mm}^2$ or larger, more preferably $0.38 \text{ mm}^2$ or larger, preferably $2 \text{ mm}^2$ or smaller, more preferably $1.7 \text{ mm}^2$ or smaller.

The areas of the embosses 80, fusion bonds 88, and fusion bonds 89 can be measured as follows.

Method for measuring areas of embosses 80 and fusion bonds 88, 89:

A leak-proof cuff 6 is cut off from the diaper 1, and a 10 mm by 10 mm piece containing the fusion bonds 89 or 88 is cut out from the upper soft region 69 or the lower joined region 68, respectively. The cut piece is subjected to image analysis using, e.g., VHX-1000 from Keyence to measure the area of every fusion bond except those with part cut off. The average of all the measurements is taken as the area of the individual fusion bonds. The area of the individual embosses 80 is measured in the same manner. That is, a 10 mm by 10 mm piece is cut out from the embossed region having the embosses 80 of the nonwoven fabric 60 forming the leak-proof cuff 6. The cut piece is analyzed using an image analyzer to measure the area of the individual embosses 80.

The ratio of the total area of the fusion bonds 89 to the surface area of the upper soft region 69 and the ratio of the total area of the fusion bonds 88 to the surface area of the lower joined region 68 are calculated in terms of the total area of the fusion bonds per unit area (10 mm by 10 mm = $100 \text{ mm}^2$) of the cut piece. The measurement is taken on at least three cut pieces, and the average of the measurement results is taken as the ratio of the total area of the fusion bonds to the area of the lower joined region. In the determination of the total fusion bond area ratios, the area measurement is taken of all the fusion bonds inclusive of those with part cut off. Similarly, the ratio of the total area of the embosses 80 to the surface area of one surface of the nonwoven fabric 60 is obtained in terms of the total area of the embosses 80 per unit area (10 mm by 10 mm = $100 \text{ mm}^2$) of the cut piece. At least three cut pieces are prepared, and the average of the measurements is taken as the ratio of the total area of the embosses 80 to the surface area of one side of the nonwoven fabric 60.

To obtain both upstanding capabilities and breathability of the leak-proof cuff 6, the shortest distance between the closest two fusion bonds 88 is preferably longer than the shortest distance between the closest two embosses 80. For the same purpose, the shortest distance between the closest two fusion bonds 89 is preferably longer than the shortest distance between the closest two embosses 80.

The shortest distance between the two closest embosses 80 of the nonwoven fabric 60 is preferably 0.4 mm or longer, more preferably 0.5 mm or longer, preferably 5 mm or shorter, more preferably 4.5 mm or shorter.

The shortest distance between the two closest fusion bonds 88 of the lower joined region 68 is preferably 3 mm or longer, more preferably 3.1 mm or longer, preferably 10 mm or shorter, more preferably 9.5 mm or shorter.

The shortest distance between the two closest fusion bonds 89 of the upper soft region 69 is preferably 3 mm or longer, more preferably 3.1 mm or longer, preferably 16 mm or shorter, more preferably 15 mm or shorter.

Materials making up constituent members of the diaper 1 are not particularly limited and can be selected from those commonly used in the art. For example, the topsheet 5 may be formed of various types of nonwoven fabric or perforated film. The leak-proof sheet 7 may be formed of sparingly liquid-permeable resin film or spun-bonded/melt-blown/spun-bonded laminated nonwoven fabric. The elastic members may be rubber treads. The outer cover-forming sheets 31 and 32 may be of various types of nonwoven fabric.

The invention has been described on the basis of its preferred embodiments, but it should be understood that the invention is not deemed to be limited thereto, and various changes and modifications can be made therein. For example, while the disposable pull-on diaper 1 according to the embodiment of Fig. 1 is of the type in which the outer cover 3 is continuous from the front portion F to the crotch portion M to the rear portion R, the disposable diaper of the invention may be of the type in which the outer cover is divided into a front panel adapted to be worn about the wearer's front and a rear panel adapted to be worn about the wearer's back, the front panel and the rear panel are connected along a pair of side seams to form a tubular shape, and the absorbent assembly is attached to the outer cover so as to bridge the front and the rear panel. The disposable diaper of the invention may be of an open-style, taped type.

The leak-proof cuff 6 of inward fold type as used in the diaper 1 (Fig. 3) may be replaced with a leak-proof cuff of outward fold type. An outward fold type leak-proof cuff has the fixed edge 62 located laterally outward of the longitudinally extending side edge of the absorbent member 4, the fold 66 located on the absorbent assembly 2, and the free edge 61 located laterally outward of the fold 66.

Industrial Applicability

The leak-proof cuff of the disposable diaper of the invention feels soft to the touch and yet has good upstanding capabilities to provide a good fit to the wearer's skin.

**Claims**

1. A disposable diaper comprising an absorbent assembly (2) comprising: a liquid permeable topsheet (5) and an absorbent member (4); and a leak-proof cuff (6) disposed along each of the sides of the absorbent assembly (2),

   the disposable diaper having a longitudinal direction, and a lateral direction perpendicular to the longitudinal direction,
   the absorbent assembly (2) having laterally opposite, longitudinally extending sides and side edges,
   the leak-proof cuff (6) having opposite lateral side edges and opposite longitudinal ends, comprising two facing nonwoven layers of nonwoven fabric (60), and having a free edge along one of the lateral side edges and a fixed edge along the other side edge,
   the leak-proof cuff (6) comprising:

      a fixed end portion (65) fixed to the absorbent assembly and located at the longitudinal ends of the leak-proof cuff;
      an elasticized free edge region (64) having an elastic member (63) arranged along the free edge (61); and
      a fold (66) located between the free edge (61) and the fixed edge (62),

   the nonwoven fabric (60) having an area located between the fixed edge (62) and the elasticized free edge region, (64) the area having a lateral compression load value measured according to the method of the description of 5 cN or less, and
   the leak-proof cuff (6) comprising:

      an upper soft region (69) comprising the two nonwoven layers and being located between the elasticized free edge region (64) and the fold (66); and
      a lower joined region (68) comprising the two nonwoven layers joined together partially or entirely and being located between the fold (66) and the fixed edge (62),

   the two nonwoven layers of the upper soft region (69) being unjoined together or having a lower peel strength than the two nonwoven layers of the lower joined region (68), the peel strength being measured according to the method of the description.

2. The disposable diaper according to claim 1, wherein the peel strength between the two nonwoven layers of the lower joined region (68) is 1.0 N/30 mm or more, and
   the two nonwoven layers of the upper soft region (69) are unjoined together or have a peel strength of less than 1.0 N/30 mm.

3. The disposable diaper according to claim 1 or 2, wherein the fixed edge (62) of the leak-proof cuff (6) is located laterally inward of the side edge of the absorbent assembly (2), and an entire of or a part of the lower joined region (68) is located laterally inward of the side edge of the absorbent assembly (2).

4. The disposable diaper according to any one of claims 1 to 3, wherein the absorbent member (4) has a flexure region in the inside in the lateral direction, wherein the flexure region is a longitudinally extending slit (44) formed through the thickness of the absorbent member or low basis weight region having a lower basis weight than the surroundings laterally inward of the side edge of the absorbent assembly.

5. The disposable diaper according to any one of claims 1 to 4, wherein the lower joined region (68) has fusion bonds formed by fusion bonding the two nonwoven layers in parts.

6. The disposable diaper according to any one of claims 1 to 4, wherein both the lower joined region (68) and the upper soft region (69) have fusion bonds (88) formed by fusion bonding the two nonwoven layers in parts.

7. The disposable diaper according to claim 5 or 6, wherein the two nonwoven layers each have embosses where constituent fibers are united by embossing in a discrete pattern in plan view, and the fusion bonds (88) have a larger area than the embosses.

8. The disposable diaper according to claim 7, wherein the two facing nonwoven layers have mutually facing surfaces and the other surfaces, and the embosses are deeper on the mutually facing surfaces than on the other surfaces.

9. The disposable diaper according to claim 7 or 8, wherein a ratio of a total area of the embosses to the surface area of one surface of the nonwoven layer is 16% or less.

10. The disposable diaper according to any one of claims 5 to 9, wherein the fusion bonds (88) are formed by heat embossing and each provide a deeper depression on the nonwoven layer facing the absorbent assembly (2) than on the other nonwoven layer.

11. The disposable diaper according to any one of claims 5 to 10, wherein a ratio of a total area of the fusion bonds (88) of the lower joined region (68) to the surface area of the lower joined region (68) is 5% or less.

12. The disposable diaper according to any one of claims 1 to 11, wherein the leak-proof cuff (6) is formed by folding a single nonwoven fabric in two.

13. The disposable diaper according to any one of claims 1 to 12, wherein the two nonwoven layers have a mean deviation of coefficient of friction (MMD) measured according to the method of the description of 0.012 or smaller.

14. The disposable diaper according to any one of claims 1 to 13, wherein the two nonwoven layers have a mean deviation of surface roughness (SMD) measured according to the method of the description of 2.2 $\mu$m or smaller

## Patentansprüche

1. Wegwerfwindel, die eine absorbierende Anordnung (2) mit einer flüssigkeitsdurchlässigen Oberschicht (5) und einem absorbierenden Element (4); und ein auslaufsicheres Bündchen (6) aufweist, das entlang jeder der Seiten der absorbierenden Anordnung (2) eingerichtet ist,

wobei die Wegwerfwindel eine Längsrichtung und eine laterale Richtung senkrecht zu der Längsrichtung aufweist,
wobei die absorbierende Anordnung (2) lateral gegenüberliegende, sich längs erstreckende Seiten und Seitenränder aufweist,
wobei das auslaufsichere Bündchen (6) gegenüberliegende laterale Seitenränder und gegenüberliegende Längsenden, umfassend zwei zugewandte Vlieslagen aus Vliesstoff (60), aufweist und einen freien Rand entlang einer der lateralen Seitenränder und einen festen Rand entlang des anderen Seitenrands aufweist,
wobei das auslaufsichere Bündchen (6) aufweist:

einen festen Endabschnitt (65), der an der absorbierenden Anordnung befestigt ist und sich an den Längsenden des auslaufsicheren Bündchens befindet;
eine elastifizierte freie Randregion (64), die ein elastisches Element (63), das entlang des freien Rands (61) angeordnet ist, aufweist; und
eine Falte (66), die sich zwischen dem freien Rand (61) und dem festen Rand (62) befindet, wobei der Vliesstoff (60) einen Bereich, der sich zwischen dem festen Rand (62) und der elastifizierten freien Randregion (64) befindet, aufweist, wobei der Bereich eine lateralen Druckbelastungswert, gemessen gemäß dem Verfahren der Beschreibung, von 5 cN oder weniger aufweist, und
wobei das auslaufsichere Bündchen (6) umfasst:

eine obere weiche Region (69), umfassend die zwei Vlieslagen und die sich zwischen der elastifizierten freien Randregion (64) und der Falte (66) befindet; und

eine untere verbundene Region (68), umfassend die zwei Vlieslagen, die zum Teil oder vollständig miteinander verbunden sind, und die sich zwischen der Falte (66) und dem festen Rand (62) befindet, wobei die zwei Vlieslagen der oberen weichen Region (69) nicht miteinander verbunden sind oder eine geringere Schälfestigkeit als die zwei Vlieslagen der unteren verbundenen Region (68) aufweisen, wobei die Schälfestigkeit gemäß des Verfahrens der Beschreibung gemessen wird.

2. Wegwerfwindel nach Anspruch 1, wobei die Schälfestigkeit zwischen den zwei Vlieslagen der unteren verbunden Region (68) 1,0 N/30 mm oder mehr beträgt und
die zwei Vlieslagen der oberen weichen Region (69) nicht miteinander verbunden sind oder eine Schälfestigkeit von weniger als 1,0 N/30 mm aufweisen.

3. Wegwerfwindel nach Anspruch 1 oder 2, wobei sich der feste Rand (62) des auslaufsicheren Bündchens (6) lateral innerhalb des Seitenrands der absorbierenden Anordnung (2) befindet und sich ein gesamter oder ein Teil der unteren verbundenen Region (68) lateral innerhalb des Seitenrands der absorbierenden Anordnung (2) befindet.

4. Wegwerfwindel nach einem der Ansprüche 1 bis 3, wobei das absorbierende Element (4) in der lateralen Richtung in dem Inneren eine Biegeregion aufweist, wobei die Biegeregion ein sich längs erstreckender Schlitz (44) ist, der durch die Dicke des absorbierenden Elements oder eine Region mit geringem Basisgewicht, die ein geringeres Basisgewicht als die Umgebung aufweist, lateral innerhalb des Seitenrands der absorbierenden Anordnung ausgebildet ist.

5. Wegwerfwindel nach einem der Ansprüche 1 bis 4, wobei die untere verbundene Region (68) Schmelzklebungen, die durch teilweises Schmelzkleben der zwei Vlieslagen ausgebildet werden, aufweist.

6. Wegwerfwindel nach einem der Ansprüche 1 bis 4, wobei sowohl die untere verbundene Region (68) als auch die obere weiche Region (69) Schmelzklebungen (88), die durch teilweises Schmelzkleben der zwei Vlieslagen ausgebildet werden, aufweisen.

7. Wegwerfwindel nach Anspruch 5 oder 6, wobei die zwei Vlieslagen jeweils Prägungen, bei denen einzelne Fasern durch Prägen in einem diskreten Muster in einer Draufsicht vereinigt sind, aufweisen und die Schmelzklebungen (88) einen größeren Bereich als die Prägungen aufweisen.

8. Wegwerfwindel nach Anspruch 7, wobei die zwei zugewandten Vlieslagen einander zugewandte Oberflächen und die anderen Oberflächen aufweisen und die Prägungen auf den einander zugewandten Oberflächen tiefer als auf den anderen Oberflächen sind.

9. Wegwerfwindel nach Anspruch 7 oder 8, wobei ein Verhältnis eines Gesamtbereichs der Prägungen zu dem Oberflächenbereich einer Oberfläche der Vlieslage 16 % oder weniger beträgt.

10. Wegwerfwindel nach einem der Ansprüche 5 bis 9, wobei die Schmelzklebungen (88) durch Heißprägen ausgebildet sind und jeweils eine tiefere Vertiefung auf der der absorbierenden Anordnung (2) zugewandten Vlieslage als auf der anderen Vlieslage bereitstellen.

11. Wegwerfwindel nach einem der Ansprüche 5 bis 10, wobei ein Verhältnis eines Gesamtbereichs der Schmelzklebungen (88) der unteren verbundenen Region (68) zu dem Oberflächenbereich der unteren verbundenen Region (68) 5 % oder weniger beträgt.

12. Wegwerfwindel nach einem der Ansprüche 1 bis 11, wobei das auslaufsichere Bündchen (6) durch Falten eines einzelnen Vliesstoffs in zwei ausgebildet ist.

13. Wegwerfwindel nach einem der Ansprüche 1 bis 12, wobei die zwei Vlieslagen eine mittlere Abweichung eines Reibungskoeffizienten (MMD), gemessen gemäß dem Verfahren der Beschreibung, von 0,012 oder kleiner aufweisen.

14. Wegwerfwindel nach einem der Ansprüche 1 bis 13, wobei die zwei Vlieslagen eine mittlere Abweichung einer Oberflächenrauheit (SMD), gemessen gemäß dem Verfahren der Beschreibung, von 2,2 $\mu$m oder kleiner aufweisen.

**Revendications**

1.  Couche jetable comprenant un ensemble absorbant (2) comprenant : une feuille supérieure perméable aux liquides (5) et un élément absorbant (4) ; et un revers étanche (6) disposé le long de chacun des côtés de l'ensemble absorbant (2),

    la couche jetable ayant une direction longitudinale, et une direction latérale perpendiculaire à la direction longitudinale,
    l'ensemble absorbant (2) ayant des côtés opposés latéralement, s'étendant longitudinalement et des bords latéraux,
    le revers étanche (6) ayant des bords latéraux opposés et des extrémités longitudinales opposées, comprenant deux couches non tissées de tissu non tissé se faisant face (60), et ayant un bord libre le long de l'un des bords latéraux et un bord fixe le long de l'autre bord latéral,
    le revers étanche (6) comprenant :

    une partie extrémité fixe (65) fixée à l'ensemble absorbant et située au niveau des extrémités longitudinales du revers étanche ;
    une région de bord libre élastique (64) ayant un élément élastique (63) disposé le long du bord libre (61) ; et
    un pli (66) situé entre le bord libre (61) et le bord fixe (62), le tissu non tissé (60) ayant une zone située entre le bord fixe (62) et
    la région de bord libre élastique, (64) la zone ayant une valeur de charge de compression latérale mesurée selon le procédé de la description de 5 cN ou moins, et
    le revers étanche (6) comprenant :

    une région souple supérieure (69) comprenant les deux couches non tissées et étant située entre la région de bord libre élastique (64) et le pli (66) ; et
    une région jointe inférieure (68) comprenant les deux couches non tissées jointes partiellement ou entièrement et étant située entre le pli (66) et le bord fixe (62),
    les deux couches non tissées de la région souple supérieure (69) n'étant pas jointes ou ayant une résistance au pelage inférieure à celle des deux couches non tissées de la région jointe inférieure (68), la résistance au pelage étant mesurée selon le procédé de la description.

2.  Couche jetable selon la revendication 1, dans laquelle la résistance au pelage entre les deux couches non tissées de la région jointe inférieure (68) est égale ou supérieure à 1,0 N/30 mm, et
    les deux couches non tissées de la région souple supérieure (69) sont non jointes ou ont une résistance au pelage inférieure à 1,0 N/30 mm.

3.  Couche jetable selon la revendication 1 ou 2, dans laquelle le bord fixe (62) du revers étanche (6) est situé latéralement vers l'intérieur du bord latéral de l'ensemble absorbant (2), et un ensemble ou une partie de la région jointe inférieure (68) est situé latéralement vers l'intérieur du bord latéral de l'ensemble absorbant (2).

4.  Couche jetable selon l'une quelconque des revendications 1 à 3, dans laquelle l'élément absorbant (4) a une région de flexion à l'intérieur dans la direction latérale, dans laquelle la région de flexion est une fente s'étendant longitudinalement (44) formée à travers l'épaisseur de l'élément absorbant ou une région de faible poids de base ayant un poids de base inférieur à celui de l'environnement latéralement vers l'intérieur du bord latéral de l'ensemble absorbant.

5.  Couche jetable selon l'une quelconque des revendications 1 à 4, dans laquelle la région jointe inférieure (68) a des liaisons par fusion formées en liant par fusion les deux couches non tissées par endroits.

6.  Couche jetable selon l'une quelconque des revendications 1 à 4, dans laquelle la région jointe inférieure (68) et la région souple supérieure (69) ont toutes deux des liaisons par fusion (88) formées en liant par fusion les deux couches non tissées par endroits.

7.  Couche jetable selon la revendication 5 ou 6, dans laquelle les deux couches non tissées ont chacune des gaufrages où les fibres constitutives sont unies par gaufrage dans un motif discret en vue en plan, et les liaisons par fusion (88) ont une surface plus grande que les gaufrages.

**8.** Couche jetable selon la revendication 7, dans laquelle les deux couches non tissées se faisant face ont des surfaces se faisant mutuellement face et les autres surfaces, et les gaufrages sont plus profonds sur les surfaces se faisant mutuellement face que sur les autres surfaces.

**9.** Couche jetable selon la revendication 7 ou 8, dans laquelle un rapport entre une zone totale des gaufrages et la zone de surface d'une surface de la couche non tissée est inférieur ou égal à 16 %.

**10.** Couche jetable selon l'une quelconque des revendications 5 à 9, dans laquelle les liaisons par fusion (88) sont formées par gaufrage à chaud et fournissent chacune une dépression plus profonde sur la couche non tissée faisant face à l'ensemble absorbant (2) que sur l'autre couche non tissée.

**11.** Couche jetable selon l'une quelconque des revendications 5 à 10, dans laquelle un rapport entre une zone totale des liaisons par fusion (88) de la région jointe inférieure (68) et la zone de surface de la région jointe inférieure (68) est inférieur ou égal à 5 %.

**12.** Couche jetable selon l'une quelconque des revendications 1 à 11, dans laquelle le revers étanche (6) est formé par le pliage en deux d'un seul tissu non tissé.

**13.** Couche jetable selon l'une quelconque des revendications 1 à 12, dans laquelle les deux couches non tissées ont un écart moyen de coefficient de frottement (MMD) mesuré selon le procédé de la description de 0,012 ou moins.

**14.** Couche jetable selon l'une quelconque des revendications 1 à 13, dans laquelle les deux couches non tissées ont un écart moyen de rugosité de surface (SMD) mesuré selon le procédé de la description de 2,2 $\mu$m ou moins

Fig. 1

Fig. 2

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011177549 A
- JP 2017023782 A
- JP 2019010348 A

- US 2005113790 A1
- JP 2016077880 A
- EP 1880700 A1

**Non-patent literature cited in the description**

- Standardization and Analysis of Hand Evaluation. Japanese Hand Evaluation and Standardization Committee (HESC), The Textile Machinery Soc. of Japan. 10 July 1980